# EUROPEAN PATENT SPECIFICATION

(11) **EP 1 775 013 B1**
(45) Date of publication and mention of the grant of the patent: **20.06.2012**
(21) Application number: 06122272.5
(22) Date of filing: 13.10.2006
(51) Int. Cl.: A61L 9/014, B01D 53/02, B01D 53/86

(54) **Air conditioner using deodorising composition with cobalt oxide**
Klimaanlage mit desodorierender Zusammensetzung mit Kobaltoxid
Climatiseur avec composition déodorante avec oxyde de cobalt

(30) Priority: 13.10.2005 JP 2005298645; 13.10.2005 JP 2005298646
(43) Date of publication of application: 18.04.2007
(73) Proprietor: Panasonic Corporation, Kadoma-shi Osaka 571-8501 (JP)
(72) Inventor: Kawazoe, Daisuke c/o Matsushita Electric Industria, Osaka-shi, Osaka 540-6319 (JP); Inoue, Yuji c/o Matsushita Electric Industria, Osaka-shi, Osaka 540-6319 (JP); Sano, Mitsuhiro c/o Matsushita Electric Industrial, Osaka-shi, Osaka 540-6319 (JP); Nakano, Koichi c/o Matsushita Electric Industrial, Osaka-shi, Osaka 540-6319 (JP)
(74) Representative: Ehlers, Jochen

(56) References cited:
- JP-A- 6 031 128
- JP-A- 2000 312 809
- JP-A- 2002 153 546
- JP-A- 2006 255 251

## Description

### BACKGROUND OF THE INVENTION

### 1. Field of the Invention

The present invention relates to a deodorizer capable of adsorbing and removing odoriferous substances contained in a gaseous body drifting within a living space such as, for example, dwelling rooms and vehicle compartments and, in particular, capable of transforming highly hazardous acetaldehyde, which is a principal component of tobacco and is one of volatile organic compounds (VOCs), into acetic acid having a less hazardous property and then removing it by adsorption. The present invention also relates to a deodorizing device and an air conditioner using such deodorizer.

### 2. Description of the Related Art

Document JP2006255251 discloses an air conditioner comprising a deodorizing material containing a cobalt oxide catalyst, which absorbs and removes aldehydes by conversion to carboxylic acid.

Hitherto, a platinum-based thermal catalyst has been widely known, which is capable of decomposing odoriferous substances into water and carbon dioxide when a platinum-containing catalyst is activated at high temperatures (200°C or higher). Also, a deodorant catalyst utilizing a complex oxide based on a transition metal containing manganese as a principal component has been proposed, in which the complex oxide containing manganese and cobalt is said to decompose 80% of acetaldehyde at 50°C (see, for example, Patent Document 1).

In addition, a photocatalytic deodorizing filter has been proposed, in which a carrier having a large specific surface area is deposited with a photocatalyst as a catalyst capable of decomposing the offensive odor at normal temperatures (see, for example, Patent Document 2 or 3).

An indoor unit of an air conditioner is generally provided with an indoor heat exchanger and a pre-filter positioned upstream of the indoor heat exchanger, and a deodorizing filter, a dust collecting unit, and a filter for eliminating bacterium and/or deactivating allergens are arranged therebetween. During the air conditioning operation of the air conditioner, relatively large dusts such as, for example, cotton dust of a few hundred microns or over are removed mainly by the pre-filter, finely divided dusts such as, for example, pollens and/or smokes of tobacco are subsequently collected by the dust collecting unit; bacterium and/or allergens are then deactivated by the allergen removal filter; and finally various stenches such as, for example, the tobacco smoke and/or stenches, which drift in a living environment, are removed by the deodorizing filter, to thereby substantially purify the air adrift within a room and to improve the living comfort. In those structures, the deodorizing filter disposed in an air flow path in the air conditioner has been prepared in a photo-regenerative deodorizing system utilizing a photo-regenerative catalyst such as, for example, titanium oxide or by the use of activated carbon (see, for example, Patent Document 4 or 5).
Patent Document 1: Japanese Laid-open Patent Publication No. H10-180108
Patent Document 2: Japanese Laid-open Patent Publication No. H06-343875
Patent Document 3: Japanese Laid-open Patent Publication No. 2003-53196
Patent Document 4: Japanese Laid-open Utility Model Publication No. S47-22566
Patent Document 5: Japanese Laid-open Patent Publication No. H01-189322

However, the conventional platinum-based catalyst and the deodorizer disclosed in Patent Document 1 require a high temperature for them to be activated and, therefore, there is a problem in that they cannot be used in association with an air conditioner or an air cleaner. When it comes to the photocatalyst, it involves numerous problems in that the deodorizing speed is low as compared with the adsorbent, the size cannot be reduced because of a light source required, the cost is high and the impact resistance is low. In the case of the deodorizing filter utilizing the photocatalyst, the deodorizing filter is required to be exposed to sunlight at all times or regularly at intervals of at least a predetermined length of time and, in the case of the use of an ultraviolet light source, replacement of a lamp is required and, because of deterioration of resin as a result of exposure thereof to ultraviolet rays of light, the freedom of design choice is limited.

Also, in the case of the use of the activated carbon, since various odoriferous gases are adsorbed to saturation by means of its physical adsorptive action, the filter is required to be replaced with a fresh one at intervals of a predetermined period of time.

Yet, in the case of the deodorizing filter of a structure, in which photocatalyst is mixed with activated carbon, care is required in such a way as to dry, for example, the filter in the sun and, in order to maintain the deodorizing performance, labor is necessarily required to perform maintenance at intervals of a predetermined period of time.

In order to provide a deodorizer capable of absorbing and removing odor generated in living space by an absorbent, converting more harmful acetaldehyde into less harmful acetic acid, and absorbing and removing by the absorbent, JP 2006-255251 (published after the priority date of the present application) proposes a deodorizer which is composed of an absorbent having physical absorption function, an oxide having catalytic function including transition elements, and a carrier carrying the physical absorbent and the catalytic oxide, wherein the catalytic oxide converts aldehydes into carbonic acids and then the physical absorbent absorbs and removes them, and the physical absorbent absorbs and removes odor generated in living space, converts more harmful acetaldehyde into less harmful acetic acid at room temperature by catalytic function of the catalytic oxide including transition elements, and the absorbent absorbs and removes it.

In order to provide a deodorizing device that exhibits sufficient deodorizing effects unlike the conventional deodorizing device that exhibits insufficient deodorizing effects, JP 2002-153546 proposes a deodorizing device which is constituted by arranging ventilating routes connected to a deodorizing section and having vents in a mat constituted of an air permeable material, and incorporating a deodorizing means and an air sucking means which sucks the air contained in the mat to the deodorizing means, in the deodorizing section.

In order to obtain outstanding deodorizing properties by fixedly attaching a deodorizing organic compound and a deodorizing inorganic compound such as a polyamine compound or a hydrazide compound to the surface of fibers constituting a filter material through a synthetic resin binder, in the filter material formed of a synthetic fiber non-woven fabric, JP 2000-312809 proposes that a filter material for separating/capturing fine solid particles afloat in the air, with a capability of reliably deodorizing the smell of ammonia or hydrogen sulfide and a tobacco odor, is formed of a synthetic fiber non-woven fabric. In this case, at least one kind of a deodorizing organic compound and one kind of a deodorizing inorganic compound selected from a polyamine compound and a hydrazide compound is fixedly attached to the surface of fibers constituting the filter medium through a synthetic resin binder.

In order to provide a method for efficiently removing a malodorous component in gas from a cooker, a heater, etc., as well as a night soil treating plant, a garbage treating plant, a food plant, a paper-pulp mill or a petrochemical plant within a wide temperature range, JP 06-031128 proposes that gas containing at least one among thiols, sulfides, disulfides, ammonia, amines and aldehydes as the principal malodorous component is brought into contact with a deodorant consisting of cobalt oxide and/or manganese oxide and zeolite. This deodorant removes the malodorous component by adsorption at a low temperature of about <=50°C, can bring the malodorous component into oxidation decomposition at a high temperature of about >=50°C and ensures an efficient deodorizing method.

### SUMMARY OF THE INVENTION

The present invention has been developed to overcome the above-described disadvantages.

An objective of the present invention is to provide an air conditioner of a type, in which the deodorizing performance can be continuously maintained.

The above objectives are accomplished by an air conditioner according to claim 1.According to this invention, odoriferous substances generated in a living space such as for example, a room or vehicle compartment can be adsorbed and, hence, removed by the adsorbent and, in particular, the hazardous aldehydes can be transformed at normal temperatures into carboxylic acids by the catalytic activity of the oxide containing Co₃O₄ as a principal component and can then be adsorbed and removed by the adsorbent.

The adsorbent may be a hydrophobic zeolite. Since zeolite having a high content of a silica component has a small polarity enough to allow the non-polar odoriferous substances to be adsorbed and the odoriferous molecules can be selectively adsorbed or desorbed without relying on the ambient humidity, a variety of odoriferous substance can be removed by adsorption.

In the case where the carrier has a honeycomb structure made of organic fibers, the carrier has a high resistance to impact, a low flow resistance and a large specific surface. Accordingly, an impact resistant deodorizer can be realized, which is capable of exhibiting a high adsorption and desorption efficiency while minimizing the pressure loss.

If the oxide is carried by at least a surface of the adsorbent, it is possible to provide a deodorizer having a high rate of transformation to the carboxylic acids since the dispersibility of the oxide having the catalytic activity can be increased and the specific surface area can also be increased and since the carboxylic acids, formed as a result of transformation, can be quickly shifted to the adsorbent due to the location of the oxide in proximity to the adsorbent, to eventually purify surfaces of the oxide.

The carboxylic acids adsorbed by the adsorbent can be desorbed by ventilation When air is caused to pass through the deodorizer including zeolite, which is an adsorbent easy to selectively adsorb or desorb, the zeolite having adsorbed to saturation can be desorbed and, hence, regenerated so that the deodorizer can be used maintenance free for a substantial period of time.

The oxide catalyst of a spinel structure is operable to oxide aldehydes to form a rock-salt structure, and subsequent oxidization of the rock-salt structure in the presence of oxygen in the air resumes the spinel structure such that repetition of those functions results in the catalysis taking place. Accordingly, selection of the spinel structure is effective to increase the capability of transforming the aldehydes to the carboxylic acids to such an extent as to permit the deodorizer to have a high ratio of transformation to the carboxylic acids.

If sodium or potassium exists in a positive ionic state, transmission and reception of electrons is so hampered as to result in lowering of the catalysis. Accordingly, when the amount of sodium or potassium components contained in the oxide is caused to be in a quantity smaller than 1 wt%, it is possible to realize the deodorizer capable of efficiently transforming the aldehydes into the carboxylic acids.

It is preferred that the aldehydes include acetaldehyde and the carboxylic acids include acetic acid. The acetaldehyde is a hazardous substance mostly contained in a substantial quantity in tobaccos and some bonding materials for construction materials and is said to be a chemical carcinogen. Accordingly, it is possible to realize the deodorizer capable of transforming the acetaldehyde into a less hazardous acetic acid, which is in turn removed by the zeolite.

An air conditioner according to the present invention includes an air intake port through which air containing odoriferous substances is introduced, an air intake means for sucking the air containing the odoriferous substances, a filter for deodorizing the odoriferous substances contained in the air sucked by the air intake means, an air return port for introducing the air, which has been deodorized by the filter, into a room or vehicle compartment, and a discharge port for discharging the odoriferous substances, which have been desorbed from the filter, to an outside of the room or vehicle compartment. The filter includes a deodorizer of the structure described above. By this construction, when the air is caused to pass through the deodorizer carrying the adsorbent, and odoriferous substances desorbed from the adsorbent, then having adsorbed to saturation, are discharged to the outside of the room or vehicle compartment, the deodorizing device can used maintenance free for a substantial period of time.

This air conditioner has a deodorizing capability that can be used maintenance free for a substantial period of time as the odoriferous substances can be desorbed from the absorbent when the heated air is caused to pass through the deodorizer carrying the adsorbent.

In this case, if a discharge means for discharging the air flowing in the air flow path within the indoor unit to an outdoor space is provided, the discharge means can be activated before or after the cleaning operation. When the heated air is caused to pass through the deodorizer carrying the adsorbent and the odoriferous substances desorbed from the adsorbent are discharged to the outside of the room, deterioration of the room environment resulting from the presence of the desorbed odoriferous substances can be relieved or prevented.

If a carrier that carries an oxide containing cobalt and having a catalytic activity is disposed in the air flow path extending from the air intake area to the air blow-off area, at least portion of odoriferous substances present inside a room can be well oxidized and, hence, decomposed to continuously deodorize the indoor odoriferous substances because of the use of the oxide that can exhibit the catalytic activity even at room temperatures.

If an adsorbent having a physical adsorption capability is carried by the carrier, the odoriferous substances are quickly removed by the physical adsorbent and, at the same time, at least portion of the odoriferous substances adsorbed by the physical adsorbent is decomposed in the presence of the oxide referred to above to permit the deodorizing performance to be maintained continuously.

If the physical adsorbent is selected to be hydrophobic zeolite that can selectively adsorb or desorb the odoriferous substances without relying on the humidity, adsorption and, hence, removal of the odoriferous substances can be accomplished with no reduction in performance even in the indoor unit of the air conditioner, which tends to become a high humid environment during the operation of the air conditioner under a cooling mode.

Although most of the various odoriferous substances adsorbed by the adsorbent having a physical adsorptive capability can be decomposed by the oxide containing cobalt and having a catalytic activity, portion of the odoriferous substances that cannot be decomposed can be accumulated on the adsorbent. Accordingly, if a cleaning function for heating the indoor heat exchanger and driving an indoor fan during an air conditioning operation of the air conditioner is provided, the carrier disposed upstream of the indoor heat exchanger can be heated during cleaning with air flowing through the carrier and, therefore, the adsorptive performance can be regenerated as the odoriferous substances adsorbed are desorbed, allowing a repeated use.

### BRIEF DESCRIPTION OF THE DRAWINGS

The above and other objectives and features of the present invention will become more apparent from the following description of preferred embodiments thereof with reference to the accompanying drawings, throughout which like parts are designated by like reference numerals, and wherein:
Fig. 1A is a schematic diagram showing an outer appearance of a deodorizer used in the air conditioner of the present invention;
Fig. 1B is a schematic enlarged diagram showing a surface area of the deodorizer of Fig. 1A;
Fig. 2 is a schematic diagram showing a deodorizing device;
Fig. 3 is a schematic diagram showing a refrigerating cycle employed in an air conditioner according to the present invention;
Fig. 4A is a schematic sectional view of an indoor unit of the air conditioner according to present invention during operation;
Fig. 4B is a view similar to Fig. 4A, but showing the indoor unit when an odor is removed from a deodorant provided therein; and
Fig. 5 is a block diagram showing the manner of how the air conditioner is controlled.

### DETAILED DESCRIPTION OF THE PREFERRED EMBODIMENTS

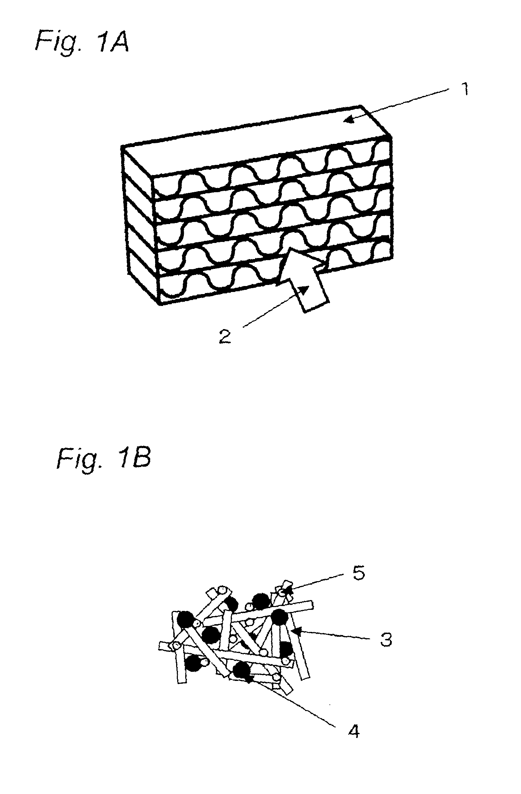

Fig. 1A illustrates a schematic diagram showing an outer appearance of a deodorizer for use in the present invention, and Fig. 1B illustrates a schematic enlarged diagram showing a surface area of the deodorizer. The deodorizer 1 is of a honeycomb structure, in which flat plates and corrugated plates are laminated alternately one above the other, each of which flat and corrugated plates is employed as a carrier 3 and made of organic fibers such as, for example, cellulose fibers or polyester fibers, represented by fibers of polyethylene terephthalate. The deodorizer 1 so constructed is capable of passing air therethrough at a low ventilation resistance in a direction indicated by 2.

The carrier 3 carries an adsorbent 4 (hereinafter referred to as a physical adsorbent 4) having a physical adsorptive function and an oxide 5 (hereinafter referred to as a catalytic oxide 5) having a catalytic activity, both of which are deposited on surfaces of the carrier 3. Specifically, the physical adsorbent 4 and the catalytic oxide 5 are coupled with and, hence, carried by the carrier 3 by means of an anchoring action or a physical or chemical binding action. A binder may be added at that time to increase such action, but if the amount of the binder added is large, it may constitute a cause of reduction of the adsorbing effect and the activity of the catalyst and, on the other hand, if the amount of the binder used is small, the adhesion with the carrier 3 will be lowered to such an extent as to result in fall-off from the carrier 3. Accordingly, the weight ratio of the physical adsorbent 4 and the binder is preferably within the range of 1:1 to 20:1 in terms of solid content. The binder may be suitably employed in the form of aluminum phosphate or colloidal silica, from which sodium and potassium components are removed as much as possible, so far as the inorganic binder is concerned, but is preferably employed in the form of an aqueous emulsion type binding material of a kind containing resinous particles of modified urethane, vinyl alcohol, ethylene, acryl and vinyl acetate, or a copolymerized resinous particles prepared from those resins, which are dispersed into water, so far as the organic binder is concerned. The organic binder can exhibit a binding action when the concentration of the resinous particles or the copolymerized resinous particles increases, as a water component is evaporated, and surfaces of those particles then start sticking together and melting together to thereby form a coating film. Thus, since the resinous film is so formed, the processability is excellent even after it has been carried by the organic fibers, but has a shortcoming in that it has a catalytic performance lower than that exhibited by the inorganic binder.

The physical adsorbent 4 may be suitably employed in the form of a material having a physical adsorption capability such as, for example, activated carbon, alumina, sepiolite, silica gel or zeolite, but zeolite is most preferred because it can exhibit selectively adsorption or desorption at a high speed. In particular, the use of hydrophobic zeolite for the physical adsorbent 4 is recommended because it has a small polarity due to the large silica/alumina ratio With it, non-polar odoriferous molecules can be adsorbed and selective adsorption and desorption of the odoriferous molecules can be accomplished without relying on the ambient humidity and, therefore, the deodorizer capable of selectively adsorbing or desorbing various odoriferous molecules can be realized. Also, when it comes to the use of zeolite or sepiolite, the one from which sodium and potassium components have been removed as much as possible is preferred. With it, the ratio of transformation from aldehydes to carboxylic acids can be increased.

The hydrophobic zeolite employed in the illustrated embodiment has a particle size within the range of 0.1 to 10pm, but it may not be limited thereto. However, the smaller the particle size, the larger the surface area for a given volume. Also, the physical adsorbent 4 may not be always limited to such a globular shape as shown in Figs. 1B, and it may be conceivable that before they are carried by the carrier 3, primary adsorbent particles gather together to form secondary adsorbent particles, which may or may not in turn gather together to form tertiary adsorbent particles. Formation of surface indentations in surfaces of the deodorizer is effective to increase the surface area per unitary volume and is, therefore, feasible.

Although in the illustrated embodiment, the deodorizer 1 has been shown and described as employing the honeycomb structure, in which flat plates and corrugated plates, each made of organic fibers, are laminated alternately one above the other, a different honeycomb structure, in which a mixture of the physical adsorbent 4 and the catalytic oxide 5 has been extruded in a grid pattern, may be employed. With it, it is possible to render the resultant honeycomb structure as a whole to be the physical adsorbent 4 and the catalytic oxide 5 with no need to use any binder and, accordingly, the deodorizer having a high adsorbent effect and a high ratio of transformation to carboxylic acids can be realized.

The catalytic oxide 5 is preferably in the form of Co₃O₄ having a spinel type crystalline structure, which is an oxide containing cobalt as a principal component. Other than it, transition metal such as, for example, Mn, Fe, Ni, Cu and/or Zn may be added to form a complex oxide having a spinel structure. Since the oxide catalyst having the spinel structure can exhibit the catalysis by cyclically repeating oxidization of aldehydes to form a rock-salt structure and subsequent oxidization of the rock-salt structure in the presence of oxygen in the air to resume the spinel structure such that repetition of those functions can result in the catalysis taking place, selection of the spinel structure is effective to increase the capability of transforming the aldehydes to carboxylic acids to such an extent as to permit the deodorizer to have a high ratio of transformation to the carboxylic acids. Also, the amount of sodium and potassium components contained in the oxide is preferably smaller than 1 wt%. This is because if sodium and potassium exist in a positive ionic state, transmission and reception of electrons will be hampered, accompanied by reduction of the catalytic activity and, therefore, the smaller amount thereof is chosen to minimize the influence which would be brought about by the transmission and reception of the electrons. Further, although the catalytic oxide employed in the practice of the illustrated embodiment has a particle size within the range of 0.1 to 10µm, the particle size may not be always limited thereto. However, the smaller the particle size, the larger the surface area for a given volume. In addition, the catalytic oxide 5 may not be always limited to such a globular shape as shown in Fig. 1B, and it may be conceivable that before they are carried by the carrier 3, primary oxide particles gather together to form secondary oxide particles, which may or may not in turn gather together to form tertiary oxide particles. Even the catalytic oxide is carried by the carrier 3 and/or the physical adsorbent 4 by means of an anchoring action or a physical or chemical binding action.

Hereinafter, the manner of how the physical adsorbent 4 and the catalytic oxide 5 are carried by the carrier 3 will now be described. Any of the physical adsorbent 4 and the catalytic oxide 5 can be carried on the carrier 3 by means of any known spray technique or any known dipping technique. However, where the carrier 3 is made of inorganic fibers, the dipping method is preferred, in which the honeycomb structure is immersed into a slurry containing both of the physical adsorbent 4 and the catalytic oxide 5 dispersed into water or solvent, together with a binder if desired. On the other hand, where the carrier 3 is made of organic fibers, both of the physical adsorbent 4 and the catalytic oxide 5 are mixed together with organic fibers added during a papermaking process, followed by papermaking to allow the physical adsorbent 4 and the catalytic oxide 5 to be deposited on the carrier 3. In the case of the dipping method, the slurry is prepared by dispersing respective powders of the physical adsorbent 4 and the catalytic oxide 5 into water or solvent as described above, in which case the average particle size of each of the physical adsorbent 4 and the catalytic oxide 5 is preferably rather small and, specifically, the average particle size of the primary particles thereof is preferably not greater than about 1µm. In addition, dispersion of the respective powders of the physical adsorbent 4 and the catalytic oxide 5 into water or solvent is preferably carried out in such a manner as to avoid aggregation or cohesion of those particles and, therefore, if the necessity may arise, a dispersant may be added thereto.

Hereinafter, some experiments conducted on the deodorizer will now be described.

### Experiment 1

Hydrophobic zeolite and tricobalt tetraoxide (Co₃O₄) (hereinafter referred to as a cobalt catalyst A) were dispersed into water, which was in turn added with a binder in the form of a sodium-free colloidal silica (hereinafter referred to as a binder A) having a solid concentration of 20 wt%, to thereby provide a slurry containing water, the hydrophobic zeolite, the cobalt catalyst A and the binder A in a ratio of 8:1:1:1 (The resultant slurry is hereinafter referred to as a slurry A).

Also, by dispersing hydrophobic zeolite and the cobalt catalyst A into water, and then adding a binder in the form of colloidal silica stabilized with sodium (hereinafter referred to as a binder B), a slurry containing water, the hydrophobic zeolite, the cobalt catalyst A and the binder B in a ratio of 8:1:1:1 was prepared. (The resultant slurry is hereinafter referred to as a slurry B.)

Again, by dispersing the cobalt catalyst A into water and then adding the binder A, a slurry containing water, the cobalt catalyst A and the binder A in a ratio of 4:1:1 was prepared. (The resultant slurry is hereinafter referred to as a slurry C.)

Thereafter, three honeycomb structures (120 x 36 x t10, 160 cells/inch²) each made of cellulose fibers were prepared. (Hereinafter, the resultant honeycomb structures are referred to as honeycombs A, B and C, respectively.) Each of the honeycombs A, B and C was immersed into the respective slurry A, B and C and, then, repeatedly dried two times at 130°C to allow the particles to be carried in a quantity of 0.1 g/cc. It is to be noted that the honeycomb B had a weight of 9.8g and implicitly contained a sodium component in a quantity of about 1wt%.

On the other hand, a honeycomb structure made of cellulose fibers and carrying 30mg of platinum and a honeycomb structure (120 x 36 x t10, 160 cells/inch²) carrying a complex oxide in a quantity of 0.05g/cc and containing manganese and cobalt in a ratio of 3:1 were prepared. (Hereinafter, those first and second mentioned honeycomb structures are referred to as honeycombs D and E, respectively.)

Samples of the three honeycombs A to C and those of two honeycombs D and E were subjected to tests, in which the concentrations of acetaldehyde at inlet and outlet were measured by means of a gas chromatography (detector FID) while they were ventilated at a concentration of 100ppm at a space velocity (hereinafter referred to as SV) of 1200. At the same time, the concentration of acetic acid at the outlet was measured by a detector tube. Results of those tests are tabulated in Table 1. It is to be noted that the experiments conducted using the honeycomb A is indicated by Example 1; the experiment conducted using the honeycomb B is indicated by Example 2; the experiment conducted using the honeycomb C is indicated by Example 3; the experiment conducted using the honeycomb D is indicated by Comparison 1; and the experiment conducted using the honeycomb E is indicated by Comparison 2.

**Table 1**

| | Initial Removal Rate (%) | Removal Rate after 1 Hour (%) | Removal Rate after 24 Hours (%) | Acetic Acid Concentration after 24 Hours (ppm) |
|---|---|---|---|---|
| Example 1 | 100 | 95 | 90 | 10 |
| Example 2 | 100 | 50 | 90 | 10 |
| Example 3 | 100 | 95 | 90 | 90 |
| Comp. 1 | 100 | 60 | 5 | 5 |
| Comp. 2 | 100 | 75 | 30 | 0 |

Table 1 above indicates that the cobalt catalyst A acts as a catalyst for modifying acetaldehyde to acetic acid and has a high activity as compared with platinum. Also, the acetic acid modified from the acetaldehyde can be adsorbed and retained by zeolite. In addition, Example 2 containing sodium has shown that the activity was once lowered, but was resumed. The reason therefor appears because the sodium becomes an ionic state, hampering transmission and reception of electrons among the cobalt catalyst A, oxygen and the acetaldehyde and it appears that formation of a compound such as, for example, sodium acetate has resulted in reduction of cations, thereby eliminating the influence brought about by the transmission and reception of the electrons.

### Experiment 2

A slurry (hereinafter referred to as a slurry D), prepared by dispersing hydrophobic zeolite and cobalt catalyst A into water, with the binder A added therein, so as to contain water, the hydrophobic zeolite, the cobalt catalyst A and the binder A in a ratio of 8:1:1:1, and a slurry (hereinafter referred to as a slurry E), prepared by dispersing activated carbon and cobalt catalyst A into water, with the binder A added therein, so as to contain water, the activated carbon, the cobalt catalyst A and the binder A in a ratio of 8:1:1:1, were prepared.

Then, two honeycomb structures (120 x 36 x t10, 160 cells/inch²), both made of cellulose fibers, were prepared (these honeycomb structures are hereinafter referred to as honeycombs D and E, respectively). Each of the honeycombs D and E was immersed into the respective slurry D and E and, then, repeatedly dried two times at 130°C to allow the particles to be carried in a quantity of 0.1 g/cc.

Samples of the two honeycombs D and E were fitted to a fan and were inserted into a 40 L container containing acetaldehyde in an adjusted concentration of 10ppm. The temperature inside the container was about 20°C. The fan was then adjusted so that it could flow gasses through the samples at a rate of 270 L/min and, 60 minutes after the start of the experiment, the concentration of acetic acid was measured with a detector tube.

After the experiment, the samples were removed out of the container and were ventilated at about 20°C for 40 minutes while the fan was operated at a place free from odoriferous substances. The above described experiment was then carried out. A series of those experiment and ventilation were repeated 10 times. Results of the experiments are shown in Table 2. It is to be noted that the experiment conducted using the honeycomb D and the experiment conducted using the honeycomb E are referred to as Example 4 and Comparison 3, respectively. It is also to be noted that the legend "N.D." appearing in Table 2 represents the limit of detection (lower than 0.05ppm).

**Table 2**

| | Acetic Acid Concentration (ppm) at First Measurement | Acetic Acid Concentration (ppm) at Tenth Measurement |
|---|---|---|
| Example 4 | N.D. | 0.1 |
| Comp. 3 | N D. | 1.0 |

Table 2 above indicates that Example 4 has shown the concentration of the acetic acid, which was low of 0.1 ppm as measured during the tenth measurement and, in contrast thereto, Comparison 3 has shown the concentration of the acetic acid, which was high of 1.0ppm as measured during the tenth measurement. This means that as compared with the activated carbon, zeolite exhibits a high regeneration rate with the acetic acid desorbed as a result of the ventilation.

In view of the foregoing, the deodorizer capable of adsorbing and, hence, removing the stench as a result of transformation of acetaldehyde to acetic acid at normal temperatures by means of the cobalt oxide catalyst and the zeolite, can be realized. Also, since the adsorbent having a physical adsorption capability can be regenerated as a result of ventilation accompanied by desorption, the deodorizer that can be used with no maintenance for a substantial period of time can be obtained.

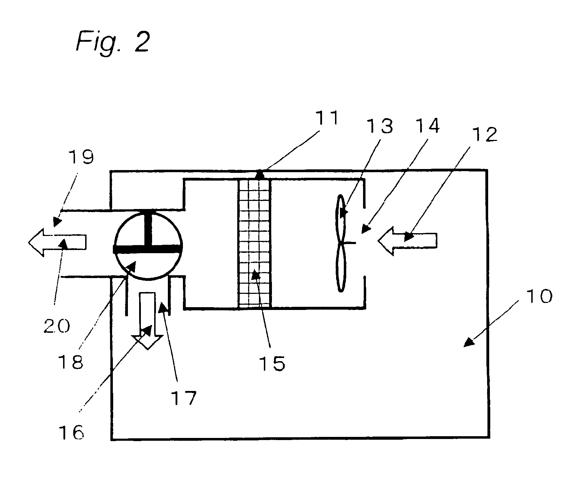

Fig. 2 illustrates a schematic diagram showing a deodorizing device. A deodorizing device 11 is disposed inside a room or vehicle compartment 10. This deodorizing device 11 includes an air intake means 13, an air intake port 14, a deodorizing filter 15, an air return port 17 through which deodorized air can be returned into the room, and a discharge port 19 through which air containing odoriferous substances can be discharged out of the room or vehicle compartment. A changeover valve 18 is disposed between the air return port 17 and the discharge port 19 for switching over therebetween. It is to be noted that the deodorizing device of the present invention can be used in the form fitted to or installed in an air conditioner or a ventilating fan.

The air intake means 13 is generally employed in the form of a sirocco fan, a turbo fan, a propeller fan, a cross-flow fan or a circulating fan, but may not be specifically limited thereto. In this embodiment, a propeller fan is used for the air intake means 13. It is to be noted that the air intake means 13 can also be used as a blower for introducing air to the filter 15.

The operation of the deodorizing device will now be described. Assuming that a stench is generated inside a room or vehicle compartment 10, the deodorizing device 11 sucks air 12, containing the stench, through the air intake port 14 by means of the air intake means 13, which air flows through the filter 15, where the stench is deodorized. The deodorized air 16 then flows through the air return port 17 and is then returned to the room 10. In the event that the filter 15 adsorbs the stench and is then saturated with the stench no longer existing inside the room or vehicle compartment 10, the direction of flow towards the discharge port 19 is selected by the changeover valve 18 and, on the other hand, the air intake means 13 is activated to ventilate so that the stench can be desorbed from the adsorbent having a physical adsorption capability, which has adsorbed the stench to a saturation level, to allow air 20 containing the stench so desorbed to be discharged to the outside of the room or vehicle compartment. Accordingly, by repeating this operation, the deodorizing device that can be used maintenance free for a substantial period of time can be realized.

### Embodiment 3

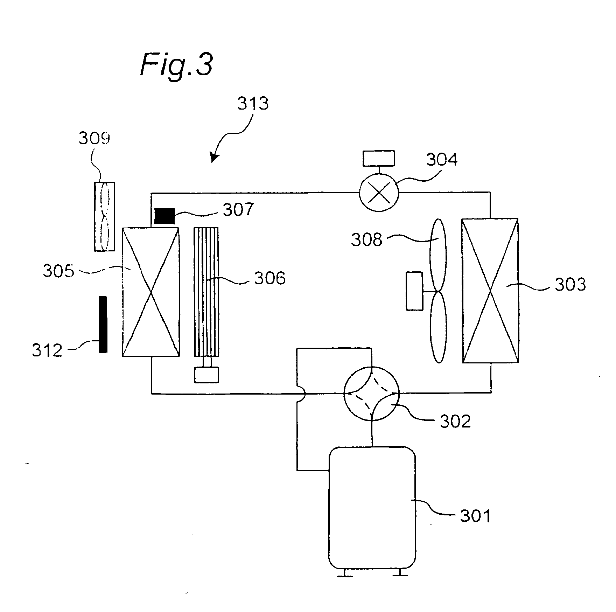

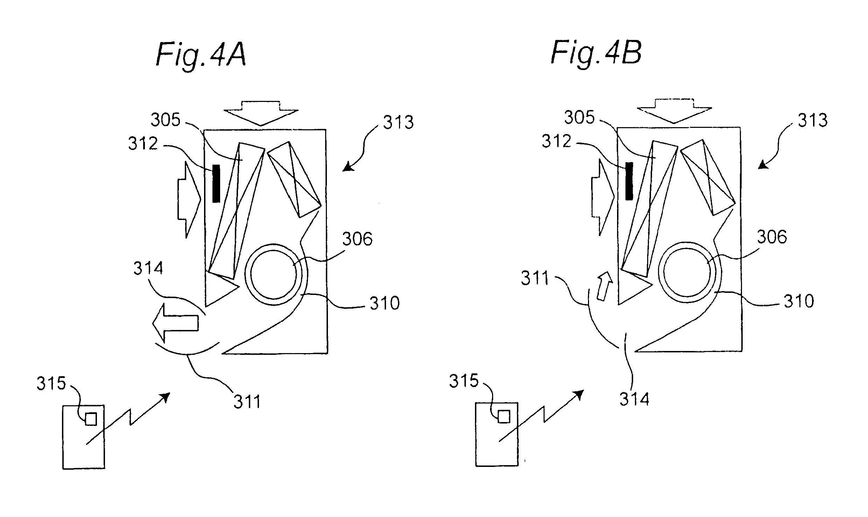

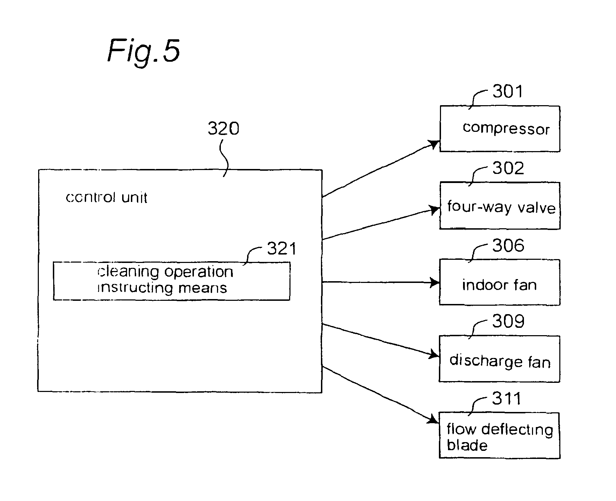

Fig. 3 illustrates a schematic diagram showing a refrigerating cycle employed in an air conditioner according to a third preferred embodiments of the present invention, Figs. 4A and 4B illustrate schematic sectional views of an indoor unit of the air conditioner, and Fig. 5 is a block diagram showing the manner of how the air conditioner is controlled.

Referring particularly to Fig. 3, the air conditioner shown therein includes a compressor 301, a four-way valve 302, an outdoor heat exchanger 303, a pressure reducing unit 304, and an indoor heat exchanger 305, all of those component parts forming a refrigerating cycle of a heat pump system. The illustrated air conditioner also includes an indoor ventilating fan 306, which forms a blower means for introducing air through the indoor heat exchanger 305, a pipeline temperature sensor 307 for detecting the temperature of a piping (not shown) forming a part of the indoor heat exchanger 305, and an outdoor fan 308 for passing air through the outdoor heat exchanger 303. A discharge fan 309 is also provided for discharging the air flowing inside an indoor unit 313 to the outside of the room or vehicle compartment through a duct (not shown) communicating between indoor and outdoor spaces. It is to be noted that the compressor 301, the four-way valve 302, the outdoor heat exchanger 303 and the outdoor fan 308 are incorporated in an outdoor unit (not shown) of the air conditioner according to this embodiment.

As best shown in Figs. 4A and 4B, the indoor unit 313 of the air conditioner includes the indoor heat exchanger 305, the indoor fan 306, the discharge fan 309, a casing 310 defining an air flow path, a flow deflecting blade 311 disposed in a blow-off opening 314, through which the air having been heat exchanged by the indoor heat exchanger 305 can be blown off from the indoor unit 313, for changing the direction of flow of the air, and a deodorizer filter 312 for removing the stench contained in the air then flowing through the air flow path. Reference numeral 315 represents a remote controller for remote controlling the operation of the air conditioner, and the user using this remote controller 315 can control the air conditioner as desired.

The air conditioning operation of the air conditioner of the structure described above will now be described in detail.

During the operation of the air conditioner under a heating mode, a refrigerant sucked in and compressed by the compressor 301 is supplied through the four-way valve 302 to the indoor heat exchanger 305, where it is condensed to liquefy. The refrigerant emerging outwardly from the indoor heat exchanger 305 is, after the pressure thereof has been reduced by the pressure reducing unit 304, introduced into the outdoor heat exchanger 303, where the refrigerant evaporates to absorb latent heat generated as a result of the evaporation of the refrigerant and is subsequently liquefied. The refrigerant having passed through the outdoor heat exchanger 303 is sucked again into the compressor 301 through the four-way valve 302.

In the air conditioner of the structure described above, the operation thereof will be described.

Fig. 4A illustrates a schematic sectional view of the indoor unit 313 of the air conditioner during air conditioning.

When the user manipulates the remote controller 315 to instruct the air conditioner to perform the air conditioning, a control unit 320 shown in Fig. 5 and built in the indoor unit 313 outputs, in response to the instruction to cause the pipeline temperature sensor 307 to detect the temperature of the indoor heat exchanger 305, control signals to the compressor 301, the four-way valve 302, the indoor fan 306 and the flow deflecting blade 311 and others if so required, with the air conditioning initiated consequently.

If at this time some stenches exist inside the indoor space, where the indoor unit 313 is installed, the odoriferous substances within the indoor space can be adsorbed and, hence, removed by the filter 312 disposed within a flow path extending from the air intake area to the blow-off opening 314.

It is to be noted that since a highly humid environment is created inside the indoor unit of the air conditioner when the latter is operated under a cooling mode, the adsorbent forming the filter 312 is most preferably in the form of a hydrophobic zeolite, but this is not specifically limited thereto.

Since the oxide containing cobalt as a principal component exhibits the catalytic activity at room temperatures, no laborious maintenance is required and the deodorizing function of the air conditioner can be continuously maintained for a substantial period of time.

In this embodiment, the oxide catalyst and the physical adsorbent are carried on the filter 312. This is particularly most advantageous in removal of the indoor odoriferous substances, which are desired to be quickly deodorized, because after the odoriferous substances have been quickly removed by the physical adsorbent, they can be decomposed by the oxide catalyst.

In the meantime, most of the various odoriferous substances found within a room can be decomposed by the catalytic oxide, but in the event that some of them are unable to be decomposed, the odoriferous substances that cannot be decomposed can be accumulated on the adsorbent without being decomposed.

In view of this, the present invention makes the indoor heat exchanger 305 heated during the halt of the air conditioning operation of the air conditioner so that a cleaning operation with the indoor fan 306 driven can be performed.

A cleaning mode of the air conditioner according to this embodiment will be described.

Referring now to Fig. 5, the control unit 320, built in the indoor unit 313, is provided with a cleaning operation instructing means 321 for instructing a cleaning operation, during which the air conditioner is operated under a heating mode, and the indoor heat exchanger 305 is heated, so that odoriferous substances deposited on the indoor heat exchanger 305 can be desorbed or the interior of the indoor unit 313 can be dried or proliferation of fungi can be suppressed by heat shocks. For this purpose, control signals if so required can be supplied from the control unit 320 to the compressor 301, the indoor fan 306, the discharge fan 309 and the flow deflecting blade 311.

When during the operation of the air conditioner, a halt command is issued from the remote controller 315, the cleaning operation instructing means 321, upon receipt of the halt command, causes a predetermined temperature T (for example, 40°C) to be set as a target temperature to be attained by the indoor heat exchanger 305 during the cleaning operation and also causes the cleaning operation instructing means 321 to control the compressor 301 and the indoor fan 306 while the pipeline sensor 307 detects the temperature of the indoor heat exchanger 305, so that the temperature of the indoor heat exchanger 305 can be adjusted to the target temperature T and the cleaning operation can take place for a predetermined length of time M (for example, 60 minutes).

Simultaneously therewith, as shown in Fig. 4B, the flow deflecting blade 311 is controlled by an odor removal operation instructing means 321 to a position, at which the air flowing outwardly from the blow-off opening 314 can be short-circuited.

As a result, the air heated by the indoor heat exchanger 305 circulates within the air flow path inside the indoor unit 313 and, as the heated air flows through the filter 312, the odoriferous substances are desorbed from the adsorbent having the physical adsorption capability, which forms the filter 312 and has adsorbed to saturation.

Accordingly, when the air conditioner is set under the heating mode as the cleaning operation each time it has been operated, the deodorizing function that can be used maintenance free for a substantial period of time can be realized in the air conditioner

Although reference has been made to the heating mode of the air conditioner which is effected as the cleaning operation that takes place automatically in response to the air conditioning halt command, effects similar to those described above can be equally obtained even when the timing at which the cleaning operation is performed is, determined at the user's will such as, for example, the start of the cleaning operation is initiated manually by the user or the cleaning operation is automatically carried out prior to the start of the air conditioning.

Thus, although the amount of the odoriferous substances generated from the filter 312 gradually increases as the cleaning operation proceeds, if a drive instruction is supplied from the odor removal operation instructing means 321 to the discharge fan 309 so that the air within the flow path inside the indoor unit 313 can be discharged through a duct to the outside, the air blown off from the blow-off opening 314 is short-circuited by the flow deflecting blade 311 and by the operation of the discharge fan 309, the stench component is discharged to the outside of the room, allowing the discharge of the odoriferous substances into the room to be lowered. Accordingly, without the comfort of the indoor environment being adversely affected, the deodorizing function, which can be used maintenance free for a substantial period of time, can be added to the air conditioner.

### [Industrial Applicability]

As hereinbefore fully described, toxic gaseous components and stenches such as, for example, tobacco smoke, smell generated during the cooking, smell from pets and formaldehyde can be adsorbed and, hence, removed to provide a clean and comfortable living environment. Accordingly, the deodorizer of the present invention can be effectively utilized not only in the air conditioner, but also in a garbage disposer, VOC decomposer, and deodorizing devices for nursing and personal care facilities. In addition, the deodorizing device of the present invention can be drivingly linked with the air conditioner and/or ventilator installed in a room or a vehicle mounted air conditioner.

## Claims

1. An air conditioner comprising:
an indoor unit having an air flow path extending from an air intake area to an air blow-off area;
an indoor heat exchanger (305);
an indoor fan (306) for supplying air of a temperature adjusted by the indoor heat exchanger (305) into a room; and
a filter for deodorizing odoriferous substances contained in air sucked therein, the filter comprising a deodorizer (1) comprising:
- an adsorbent (4) having a physical adsorption capability;
- an oxide (5) containing cobalt and having a catalytic activity; and
- a carrier (3) for carrying the adsorbent (4) and the oxide (5); and
the indoor heat exchanger (305), the indoor fan (306) and the carrier (3) being disposed in the air flow path;
**characterized by**
a control unit (320) configured to heat the indoor heat exchanger (305) during a halt of the air conditioning operation of the air conditioner, so that a cleaning operation with the indoor fan (306) driven can be performed.

2. The air conditioner as claimed in claim 1, further comprising an adsorbent (4) having a physical adsorption capability and being carried by the carrier (3).

3. The air conditioner as claimed in claim 1,
wherein the oxide (5) transforms aldehydes to carboxylic acids, and the adsorbent (4) removes the carboxylic acids by adsorption.

4. The air conditioner as claimed in Claim 2 or 3, wherein the adsorbent (4) is hydrophobic zeolite.

5. The air conditioner as claimed in claim 1, further comprising a flow deflecting blade (311) disposed in the blow-off area to change a direction of flow of the air blown out from the indoor unit, wherein the flow deflecting blade (311) is controlled to direct the air blown out from the indoor unit towards the air intake area during cleaning operation.

6. The air conditioner as claimed in any one of Claims 1 to 5, further comprising a discharge means (309) for discharging the air flowing in the air flow path within the indoor unit to an outdoor space, the discharge means (309) being activated during or after the cleaning operation.

## Patentansprüche

1. Klimaanlage, enthaltend:
eine Inneneinheit, die einen Luftströmungsweg aufweist, der sich von einem Lufteinlassbereich zu einem Luftausblasbereich erstreckt;
einen Innen-Wärmetauscher (305);
ein Innengebläse (306) zum Zuführen von Luft mit einer Temperatur, die durch den Innen-Wärmetauscher (305) eingestellt wird, in einen Raum; und
einen Filter zum Desodorieren riechender Substanzen, die in der angesaugten Luft enthaltend sind, in demselben, wobei der Filter ein desodorierendes Mittel (1) enthält, enthaltend:
- ein Adsorptionsmittel (4), das eine physikalische Adsorptionsfähigkeit aufweist,
- ein Oxid (5) das Kobalt enthält und eine katalytische Aktivität aufweist; und
- einen Träger (3) zum Tragen des Adsorptionsmittels (4) und des Oxids (5); und
wobei der Innen-Wärmetauscher (305) das Innengebläse (306) und der Träger (3) im Luftströmungsweg angeordnet sind;
**gekennzeichnet durch**
eine Steuereinheit (320), die dafür konfiguriert ist, den Innen-Wärmetauscher (305) während einer Pause des Klimatisierungsbetriebs der Klimaanlage aufzuheizen, so dass ein Reinigungsvorgang mit angetriebenem Innengebläse (306) durchgeführt werden kann.

2. Klimaanlage nach Anspruch 1, ferner ein Adsorptionsmittel (4) enthaltend, das eine physikalische Adsorptionsfähigkeit aufweist und vom Träger (3) getragen wird.

3. Klimaanlage nach Anspruch 1,
wobei das Oxid (5) Aldehyde zu Carboxylsäuren transformiert und das Adsorptionsmittel (4) die Carboxylsäuren durch Adsorption entfernt.

4. Klimaanlage nach Anspruch 2 oder 3, wobei das Adsorptionsmittel (4) hydrophobes Zeolith ist.

5. Klimaanlage nach Anspruch 1, ferner einen Strömungsablenkflügel (311) enthaltend, der im Ausblasbereich angeordnet ist, um eine Strömungsrichtung der aus der Inneneinheit ausgeblasenen Luft zu ändern, wobei der Strömungsablenkflügel (311) so gesteuert wird, dass er die aus der Inneneinheit ausgeblasene Luft während des Reinigungsvorgangs in Richtung zum Lufteinlassbereich lenkt.

6. Klimaanlage nach irgendeinem der Ansprüche 1 bis 5, ferner ein Ausgabemittel (309) enthaltend zum Ausgeben der im Luftströmungsweg innerhalb der Inneneinheit strömenden Luft an einen Außenraum, wobei das Ausgabemittel (309) während des Reinigungsvorgangs oder nach demselben aktiviert ist.

## Revendications

1. Climatiseur comprenant :
une unité intérieure ayant une voie d'écoulement d'air s'étendant d'une zone d'admission d'air jusqu'à une zone de soufflage d'air ;
un échangeur de chaleur intérieur (305) ;
un ventilateur intérieur (306) pour délivrer un air à une température ajustée par l'échangeur de chaleur intérieur (305) dans une pièce ; et
un filtre pour désodoriser des substances odorantes contenues dans l'air aspiré dans celui-ci, le filtre comprenant un désodoriseur (1) comprenant :
- un adsorbant (4) ayant une capacité d'adsorption physique ;
- un oxyde (5) contenant du cobalt et ayant une activité catalytique ; et
- un support (3) pour supporter l'adsorbant (4) et l'oxyde (5) ; ; et
l'échangeur de chaleur intérieur (305), le ventilateur intérieur (306) et le support (3) étant disposés dans la voie d'écoulement d'air ;
**caractérisé par**
une unité de commande (320) configurée pour chauffer l'échangeur de chaleur intérieur (305) pendant un arrêt de l'opération de climatisation du climatiseur, de manière à ce qu'une opération de nettoyage avec le ventilateur intérieur (306) entraîné puisse être exécutée.

2. Climatiseur selon la revendication 1, comprenant en outre un adsorbant (4) ayant une capacité d'adsorption physique et étant supporté par le support (3).

3. Climatiseur selon la revendication 1,
dans lequel l'oxyde (5) transforme des aldéhydes en acides carboxyliques, et l'adsorbant (4) élimine les acides carboxyliques par adsorption.

4. Climatiseur selon la revendication 2 ou 3, dans lequel l'adsorbant (4) est une zéolite hydrophobe.

5. Climatiseur selon la revendication 1, comprenant en outre un déflecteur d'écoulement (311) disposé dans la zone de soufflage pour changer un sens d'écoulement de l'air soufflé à partir de l'unité intérieure, dans lequel le déflecteur d'écoulement (311) est commandé de façon à diriger l'air soufflé à partir de l'unité intérieure vers la zone d'admission d'air lors d'une opération de nettoyage.

6. Climatiseur selon l'une quelconque des revendications 1 à 5, comprenant en outre un moyen de décharge (309) pour décharger l'air s'écoulant dans la voie d'écoulement d'air au sein de l'unité intérieure jusqu'à un espace extérieur, le moyen de décharge (309) étant activé pendant ou après l'opération de nettoyage.
